# EUROPEAN PATENT SPECIFICATION

(11) **EP 1 143 992 B1**
(45) Date of publication and mention of the grant of the patent: **12.01.2005**
(21) Application number: 00908420.3
(22) Date of filing: 27.01.2000
(51) Int. Cl.: A61K 38/06, A61K 9/127

(54) **THERAPEUTIC COMPOSITIONS CONTAINING GLUTATHIONE ANALOGS**
GLUTATHIONEANALOGE ENTHALTENDE THERAPEUTISCHE ZUSAMMENSETZUNGEN
COMPOSITIONS THERAPEUTIQUES A BASE D'ANALOGUES DE GLUTATHIONE

(30) Priority: 27.01.1999 US 238530
(43) Date of publication of application: 17.10.2001
(73) Proprietor: TELIK, INC., Palo Alto, CA 94304 (US)
(72) Inventor: REDELMEIER, Thomas, Vancouver, British Columbia V6N 1Y8 (CA); KAUVAR, Lawrence, M., San Francisco, CA 94115 (US); LUM, Robert, T., Palo Alto, CA 94306 (US); LYTTLE, Matthew, H., Point Reyes Station, CA 94956 (US); MACSATA, Robert, W., Emeryville, CA 94608 (US); SCHOW, Steven, R., Redwood Shores, CA 94065 (US); VILLAR, Hugo, O., Newark, CA 94560 (US); KOZLOWSKI, Michael, R., Palo Alto, CA 94303 (US)
(74) Representative: Sexton, Jane Helen
(86) International application number: PCT/US2000/002297
(87) International publication number: WO 2000/044366

(56) References cited:
- WO-A-95/08563
- WO-A-96/40205
- CIACCIO P J ET AL: "MODULATION OF DETOXIFICATION GENE EXPRESSION IN HUMAN COLON HT29 CELLS BY GLUTATHIONE-S-TRANSFERASE INHIBITORS" MOLECULAR PHARMACOLOGY,US,BALTIMORE, MD, vol. 48, no. 4, 1 October 1995 (1995-10-01), pages 639-647, XP000601686 ISSN: 0026-895X
- MORGAN A S ET AL: "ISOZYME-SPECIFIC GLUTATHIONE S-TRANSFERASE INHIBITORS POTENTIATE DRUG SENSITIVITY IN CULTURED HUMAN TUMOR CELL LINES" CANCER CHEMOTHERAPY AND PHARMACOLOGY,DE,SPRINGER VERLAG, BERLIN, vol. 37, no. 4, 1996, pages 363-370, XP000601657 ISSN: 0344-5704
- SCHULTZ M ET AL: "INHIBITORS OF GLUTATHIONE S-TRANSFERASES AS THERAPEUTIC AGENTS" ADVANCED DRUG DELIVERY REVIEWS,NL,AMSTERDAM, vol. 26, no. 2/03, 1 July 1997 (1997-07-01), pages 91-104, XP002056137 ISSN: 0169-409X

## Description

### Technical Field

The invention relates to compositions, formulations and methods of certain glutathione analogs which interact with at least one glutathione S-transferase. The invention further relates to lipid formulations of a glutathione analog and to their process of manufacture. The lipid formulations overcome the insolubility problem of the drug when administered parenterally and decrease its toxic effects. Finally, the invention is directed to modulation of hematopoiesis in bone marrow or blood and to other useful responses provided by such compositions and formulations.

### Background and Disclosure of the Invention

The side effects of chemotherapeutic agents used in the treatment of malignancy and other indications are well known. Among these side effects are alterations in the levels of various blood cells, including neutrophils, platelets and lymphocytes. The results of these effects can be neutropenia, thrombocytopenia and general immune suppression. These side effects are not only unpleasant, but they also restrict the efficacy of cancer therapy and place the subject at serious risk of infection and uncontrolled bleeding.

At the present time, there is little practical remediation for these effects. Typically, the approaches are supportive care, large doses of antibiotics or the administration of growth factors. The administration of growth factors, such as granulocyte colony-stimulating factor (GCSF), granulocyte macrophage colony-stimulating factor (GMCSF), and more newly developed factors such as megakaryocyte growth and development factor (MGDF) and thrombopoietin (TPO) are costly. In addition, they have their own associated negative side effects.

The problems related to current approaches for managing the side effects of chemotherapy and otherwise dealing with suppression of hematopoiesis are solved, at least in part, by the biological activity of certain simple tripeptide compounds which are inhibitors of the various isoenzymes of glutathione S-transferase.

As with many small peptide-based compounds, the formulation of such compounds plays a substantial role in their solubility and efficacy. It is known that lipid formulations (Liposomes as Drug Carriers, ed. G. Gregoriadis, John Wiley and Sons, 1988) and emulsions or lipid microspheres (Cummings, J. et. al. Expert Opin. Ther. Pat. (1998), 8(2), 153; Takenaga, M. Adv. Drug Delivery Rev. (1996), 20(2,3), 209; Yamaguchi, T. Adv. Drug Delivery rev. (1996), 20(2,3), 117; Mizushima, Y. EP 0432697A2; Kraft, M. WO 98/05301) can be used as suitable pharmaceutical carriers that enhance solubility, modulate pharmacokinetic behavior, modify biodistribution, and protect compounds from enzymatic degradation. Increasing the lipophilicity of the compound oftentimes aids in the formulatability of compounds in emulsions and liposomes. Esters containing longer carbon chains or other lipophilic groups can, thus, be used to tailor compounds to maximize solubility.

A liposome is a completely closed lipid bilayer membrane which defines a closed aqueous compartment. Liposomes are microscopic delivery vesicles made, in part, from phospholipids which form closed, fluid-filled spheres when mixed with water. Liposomes may be either unilamellar, comprised of one lipid bilayer membrane, or multilamellar, comprised of more than bilayer. Liposomes have the potential of providing controlled release of the administered drug over an extended period of time and of reducing toxic side effects of the drug by limiting the free concentration of the active agent in the bloodstream. Liposomes can also alter the tissue distribution and uptake of drugs, and the altered tissue distribution can significantly increase the therapeutic effectiveness of the drug.

WO 96/40205 and WO 95/08563, disclose tripeptide compounds which are analogs of glutathione. They are generally inhibitors of glutathione S-transferase activity, and the various compounds contained in this group show diverse specificities with respect to glutathione S-transferase isoenzymes. Disclosed in these patents are symmetrical esters of 1 to 10C units, with the preferred embodiment as the diethyl ester.

### Summary of the Invention

The invention provides lipid formulations containing compounds, which are useful in modulating hematopoiesis generally and as aids to the chemotherapeutic treatment of tumors. The invention provides lipid formulations and methods to produce such formulations. In one aspect, the invention is directed to a lipid formulation containing a compound that is
(i) a diester of a compound of formula A 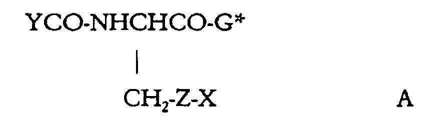 wherein:
   each ester is 1-25C;
   YCO is γ-glu or β-asp;
   G* is phenylglycine;
   Z is CH₂, O or S; and
   X is a hydrocarbon radical which is alkyl (6-8C), benzyl, or naphthyl;
   or a pharmaceutically acceptable salt thereof; or
(ii) a compound of formula I 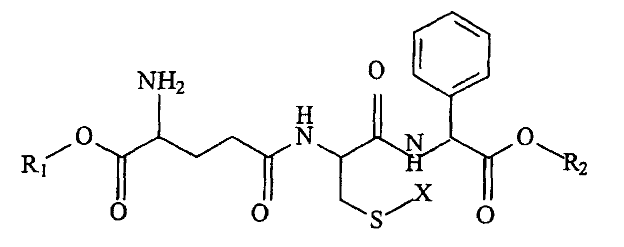 wherein:
   R₁ and R₂ are each independently linear or branched alkyl (1-25C), cycloalkyl (6-25C), heterocycle (6-20C), ether or polyether (3-25C), or R₁-R₂ together have 2-20 C atoms and form a macrocycle with the remainder of formula I; and
   X is as defined above for formula A;
or a pharmaceutically acceptable salt thereof;
wherein the lipids of said lipid formulation comprise egg phosphatidylcholine and egg phosphatidylglycerol in a ratio of 0.75-1.25:0.75-1.25 by weight.

In another aspect the present invention provides the use of the lipid formulation in the manufacture of a medicament for the modulation of hematopoiesis and protection against the destructive effects of chemotherapy.

Prior to the present invention, efforts were made to increase the bioavailability of the compounds described above by producing higher alkyl esters, where at least one of R1 or R2 is a higher alkyl (at least 6C) group. This proved to be a more difficult and expensive process, however. The inventors subsequently found that by using lipid formulations, in particular, liposome formulations, they could enhance the uptake of the compounds via appropriate targeting and, therefore, increase bioavailability without having to resort to the aforementioned difficult, expensive production of a higher alkyl esters.

Lipid formulations or compositions in accordance with the invention include liposomes, lipid microspheres, and lipid emulsions. Lipid microspheres are spherical) particles composed of a lipid core and/or matrix containing the compounds of the invention. Liposomes are phospholipid vesicles consisting of lipid layer(s) bounding an aqueous compartment containg the compounds of the invention.

The compounds of formulae A or I have been shown to have limited solubility in aqueous solutions, especially in high osmotic solutions. These compounds formulated in lipids are shown to have greater solubility when placed in biological medium. These compounds have further been shown, when formulated in lipid microspheres, to have greater resistance to proteases, and an increased half-life in the presence of blood.

In one aspect, the present invention provides a method for the preparation of a lipid medium of droplets comprising of a core of compounds of formulae A or I coated with a pharmaceutically acceptable oil. Thus, the, invention provides methods for the preparation of a lipid-based formulation.

In another aspect, the invention provides a method for the preparation of a. phospholipid-based liposome wherein compounds of formulae A or I are found associated'with the liposome.

In another aspect, the invention is directed to a process to produce phospholipid-based liposomes containing compounds of formulae A or I.

In another aspect, the invention is directed to the use of a lipid formulation of the invention in the manufacture of a medicament for the modulation of hematopoiesis and protection against the destructive effects of chemotherapy.

In other aspects, the invention is directed to lipid formulations containing a diester of a compound of formula A or a compound of formula I for promoting the production of neutrophils, platelets and lymphocytes, restoring damaged bone marrow, protecting bone marrow from cytotoxic therapy, and exerting a protective effect against neutropenia, thrombocytopenia,. lymphocytopenia and anemia caused by chemotherapy, infection or hematological diseases and for the expansion of cell populations in the course of bone marrow transplantation. A preferred embodiment is the inclusion of low alkyl esters in lipid formulations. The invention is further directed to the use of compounds of the invention as tumor-specific chemo- or radiosensitizers, thus potentiating the effect of treatment, and as generalized chemoprotectants. Another aspect of this invention is directed to a general immune stimulant for the treatment of anemia, infections, and the like.

Lipid formulations containing the compounds of formulae A or I which'may be produced in the following manner: (I) drug and lipid are dissolved in an alcohol/water co-solvent; (ii) the mixture is rapidly diluted into a buffer to form liposomes; (iii) the liposome preparation is extruded through one or more stacked filters to generate unilamellar vesicles of a defined'size; and (iv) lyophilized.

### Detailed Description of the Invention

Many of the compounds of formulae A and I are useful to inhibit the activity of at least one isoenzyme subclass of the glutathione S-transferase isoenzymes. Compositions of these compounds also modulate hematopoiesis in bone marrow, even in the presence of agents which normally would destroy a large percentage of the cells needed to sustain hematopoeisis. In addition, these compositions exhibit other helpful effects on bone marrow and blood cells.

Compounds of the invention of formulae A or I are present as diesters. The ester forms are generally those of alkyl (1-25C); alkenyl (1-25C); and arylalkyl (1-25C) alcohols and amines. In particular, any of the diesters may include those with a first ester group having 1C atom and the second ester group having 1C, 2C, 3C, 4C, 5C, 6C, 7C, 8C, 9C, 10C, 11C, 12C, 13C, 14C, 15C, 16C, 17C, 18C, 19C, 20C, 21C, 22C, 23C, 24C or 25C atoms. Similarly, they may include those having any of the foregoing second ester groups in combination with a first ester group of 1C, 2C, 3C, 4C, 5C, 6C, 7C, 8C, 9C, 10C, 11C, 12C, 13C, 14C, 15C, 16C, 17C, 18C, 19C, 20C, 21C, 22C, 23C, 24C or 25C atoms. Such groups may be straight chain or, for those with sufficient number of atoms, have one or more branches. Particularly, at least one of the ester groups may have any one of from 10 to 25C atoms. Typical esters useful in the invention include diolelyl, palmityl/ethyl, lauryl/ethyl, .dilauryl, stearyl/ethyl and the like.. Especially preferred are the palmityl/ethyl forms of the compounds of formulae A or I where is benzyl. It will be understood that such groups, and the remainder of formulae A or I, may have one or more substituents, provided such substituents do not prevent the compound from exhibiting a positive pharmaceutical effect (such as at least any one of the effects described herein). Such substituents may include one or more (such as two or three) halogens or hydroxy,

A particular diester of formulae A or I (for example, a diester of formula I) may have a greater lipophilicity than a corresponding diethyl ester. This may be particularly beneficial when the compound is formulated in a liposomal composition containing the compound. A diester may be selected which exhibits enhanced potentiation of chlorambucil cytotoxicity on human cells in comparison with the corresponding free di-acid form of the compound, as is described in Example 1 of U.S. Patent No. 5,955,432 or which exhibits enhanced uptake by human cancer cells such as HT-29 (human colon adenocarcinoma) suspended in serum-free medium versus the corresponding free di-acid form of the compound (as described in Example 1 of the foregoing patent). A diester may be selected which provides enhanced differentiation of mouse or rat bone marrow in comparison with the corresponding free di-acid form of the compound (as described in Example 9 of the foregoing patent).

It is evident that the tripeptides of the invention contain one or more chiral centers. The designations set forth above are directed to the genus of diastereomers which result from the presence of these chiral centers. The preferred amino acids of the invention are of L-configuration.

The compositions of the invention have several properties which make them useful as adjuncts to chemotherapy and other indicators. In the first, they modulate hematopoiesis in bone marrow, the destruction of which is a common side effect of chemotherapeutic agents. Secondly, they usually inhibit at least one class of the GST isoenzymes, including the π subclass, which is particularly prevalent in tumor cells. Finally, the compounds of formulae A or I directly potentiate the effect of chemotherapeutic agents in the destruction of tumor cells. This combination of qualities makes the compounds of the invention useful both as hematopoiesis potentiating agents directly and to ameliorate the negative effects of chemotherapeutic protocols, as well as to enhance the toxic effect to the target cells. When formulated for use in vivo or in contact with intact cells, the compounds of formulae A or I will be supplied as the diesters.

Formulations for administration will employ standard methods such as those described in Remington's Pharmaceutical Sciences, (Mack Publishing Company, Easton, PA.) and in Liposome Technology, Volume III. Targeted Drug Delivery and Biological Interaction (1984, CRC Press, Inc., Boca Raton, Florida), as well as specialized formulations as described. Particularly useful formulations for compounds of formulae A or I are lipid compositions. Lipid compositions include lipid emulsions (such as aqueous lipid emulsions), liposomes, and lipid microspheres. Formulations in lipid compositions contain phosphatidylcholine and phosphatidylglycerol.

A preferred composition is that of a compound of formula I where R1 and R2 are ethyl, X is benzyl, and formulated in a mixture of egg phosphatidylcholine (EPC) and egg phosphatidylglycerol (EPG).

The lipid formulation may comprise a 3:1 to 6:1 ratio of lipid to compound of formulae A or I-. Preferably, the lipid formulation may comprise by weight, 2:2 parts egg phosphatidylcholine (EPC): egg phosphatidylglycerol (EPG), 1 part compound of formulae A or I, and 7 parts sucrose. Most preferably, the lipid formulation may comprise by weight, 2 parts egg phosphatidylcholine (EPC), 2 parts egg phosphatidylglycerol (EPG), 1 part compound of formulae A or I, and 7 parts sucrose. Further, additional agents such as citrate buffer may be added to control for pH. The net charge of the lipid formulation may range from negative to neutral. Preferably, the net charge of the lipid formulation is negative. The process of preparation of the lipid/compound formulation is preferably by use of an extruder, followed by filtration to generate unilamellar vesicles of a defined size of about 50-2000 nm. More preferably, the unilamellar vesicles generated will be about 50-1000 nm in size. Most preferably, the unilamellar vesicles generated will be 400-600 nm in size. The liposomes may be lyophilized to dryness and reconstituted with a pharmaceutically acceptable diluent.

Preferably, the degree of encapsulation of drug in liposome is greater than 50%, more preferably more than 80%, and final lyophilization may increase viscosity and vesicle size (75-600 nm). Most preferably, the degree of encapsulation of drug to liposome is about 95%.

### Administration and Use

By "modulating hematopoiesis in bone marrow or peripheral blood" is meant altering the rate of blood cell formation as measured by the capacity to form colonies or differentiated cells. Differentiated cells include neutrophils, platelets, red blood cells, lymphocytes, macrophage, granulocytes, granulocyte-macrophage and the like. As used in the present application, "modulating hematopoiesis in bone marrow or peripheral blood" refers to the ability of bone marrow or blood treated with the compositions of the invention to exhibit colony formation or generation of differentiated cells at a level different from that of untreated bone marrow. Similarly, fractions of bone marrow or peripheral blood which contain suitable progenitors will exhibit this effect. It should be noted that, as used herein, "peripheral blood" specifically includes cord blood.

In general, when agents are employed which typically have destructive effects on bone marrow or on hematopoiesis in blood, the compositions of the invention exert a protective effect. By "protective effect" is meant that the resultant damage to the bone marrow or blood is less when the composition is administered than when it is not.

There are a number of situations in which the protective effect of the compositions of the invention are useful. These include instances where irradiation has resulted, or may result prospectively, in negative effects, instances where a subject is immunocompromised for any reason, instances wherein a subject exhibits damage to the kidneys, and instances wherein the subject has been subjected to chemotherapy. In addition, the compositions of the invention may be used in transplantation settings to increase the number of cells in the bone marrow of a donor; typically, in this case the composition may be administered *in vivo* or *ex vivo.* In this setting also, the compositions of the invention promote the movement of progenitor cells into the peripheral blood of the donor which, thus, improves the recovery of peripheral white blood cell numbers in this donor; similarly, the compositions of the invention may improve the recovery of peripheral white blood cell numbers in the recipient. In general, the compositions will improve the expansion and promote the eventual engraftment of transplanted cells after exposure to the compositions of the invention *in vivo* or *ex vivo.* The compositions of the invention can be used directly in the recipient to hasten recovery.

The compositions of the invention can be used either *in vitro* or *in vivo.* For example, these compositions can be employed to expand or otherwise modulate hematopoietic cells in bone marrow prior to allogeneic or xenogeneic transplants. Treatment of subjects using ex vivo techniques whereby expansion of relatively undifferentiated cells from the bloodstream is effectuated may also be employed. The compositions of the invention can also be formulated for in vivo administration.

When ex vivo administration is employed, either bone marrow or peripheral blood (including cord blood) or both can be directly contacted with the invention compositions, or fractions of these materials may be treated so long as the fractions contain suitable target progenitor cells.

The compounds may be formulated for injection, for oral administration, or for alternative methods of administration such as transmucosal or transdermal administration. Injection can be intravenous, intraperitoneal, intramuscular, or by any other conventional route.

The percentage of active ingredient compound (or mixture of compounds) in the formulation may vary over a wide range, such as from about 0.5% w/w to about 95% w/w or up to 100%. The preferred percentage of active ingredient will be dependent on the nature of the formulation per se. Thus, a "pharmaceutical composition" of a compound of the present invention may, for example, contain only the compound or the compound and other (preferably non-toxic) components. The preferred percentage of active ingredient will be dependent on the nature of the formulation per *se.* In addition, the compositions of the invention may be mixed with or used in addition to other beneficial agents such as immunostimulants or growth factors.

The dosage required depends on the nature of the subject, the nature of the condition, the manner of administration, and the judgment of the attending physician or veterinarian. Suitable dosage ranges are adjusted according to these parameters. In general, typical doses per patient will be in the range of 0.1-100 mg/kg per day for 10-40 days, more preferably 1-10 mg/kg per day for 14-28 days. These ranges are merely illustrative, and the correct dosage optimization can be determined by routine methods.

If the invention compositions are administered as protective agents with regard to chemotherapeutic treatment, the timing of administration may also be relevant. The timing will, however, depend on the nature of the chemotherapeutic agent used. It is clearly within routine skill to determine appropriate timing for the specific chemotherapeutic agent employed.

Diesters are chosen which preferably have a higher number of C atoms in at least one ester group and which exhibit a lower rate of hydrolysis and a higher half-life, in animal or human plasma, for example, C16/C2 versus the corresponding C2/C2 diester). Similarly, a diester may be chosen having a higher number of C atoms in at least one ester group (such as C16/C2 diester) and which exhibits enhanced modulation of hematopoiesis of animal or human cells in comparison with the corresponding diester having a lower number of ester group C atoms (such as the C2/C2 diester).

The Examples which follow serve to illustrate this invention. Further examples are provided in above-referenced U.S. Patent No. 5,955,432 for particular compounds, and other compounds described herein can be used in an analogous manner. The Examples are in no way intended to limit the scope of this invention, but are provided to show how to make and use the compounds of this invention. In the following Examples, all temperatures are in degrees Centigrade, and RT. indicates room temperature. Glutathione analogues may be prepared by typical synthetic organic procedures well known to the art, such as those described in US 5,786,336. In some cases, protective groups may be introduced and finally removed. Suitable protective groups for amino, hydroxyl, carboxyl groups are described in Greene, et al. "Protective Groups in Organic Synthesis," Second Edition, John Wiley and Sons, New York, 1991. Activation of the carboxylic acid can be achieved by using a number of different reagents as described in Larock, "Comprehensive Organic Transformations", VCH Publishers, New York, 1989.

Preparation of lipid emulsions and liposomes are accomplished by techniques well known to the art. Here, the soybean oil used as a glyceride is purified soybean oil, having a high purity (purity at least 99.9% as triglyceride, diglyceride and monoglyceride). The phospholipid is purified, such as egg-yolk lecithin or soybean lecithin, which may be prepared by a separation method by means of a usual organic solvent. Generally, pre-determined amounts of soybean oil, lipid, a glutathione analogue, and other additives as mentioned above are mixed and heated to form a solution and subjected to homogenizing treatment by means of a usual homogenizer (such as a pressurized jet type homogenizer or an ultrasonic homogenizer) at a temperature of 25 to 90° to obtain a water-in-oil dispersion. Then a necessary amount of water is added, and the mixture is again homogenized by means of the above homogenizer to convert it into an oil-in-water type emulsion, whereby the emulsion of the present invention is prepared. Depending upon the convenience for the production, additives such as a stabilizer or isotonic agent may be added after formulation of the emulsion.

Liposomes may be prepared according to methods known to the art and described in Liposome Technology, Volume III, Targeted Drug Delivery and Biological Interaction (1984, CRC Press, Inc., Boca Raton, Florida). Liposomes may also be produced by dissolving lipid and the compounds of formulae A or I in an alcohol/water solvent, rapidly diluting the mixture in an appropriate buffer, extruding the product through at least one filter, and subsequently lyophilizing the filtrate.

### Example 1

### Preparation of Glutathione Analogues

L-Glutamic acid **1,** is protected using di-t-butyldicarbonate under basic conditions. Compound **2** is then cyclized to give the oxazolidinone 3 through the reaction of paraformaldehyde, and a catalytic amount of 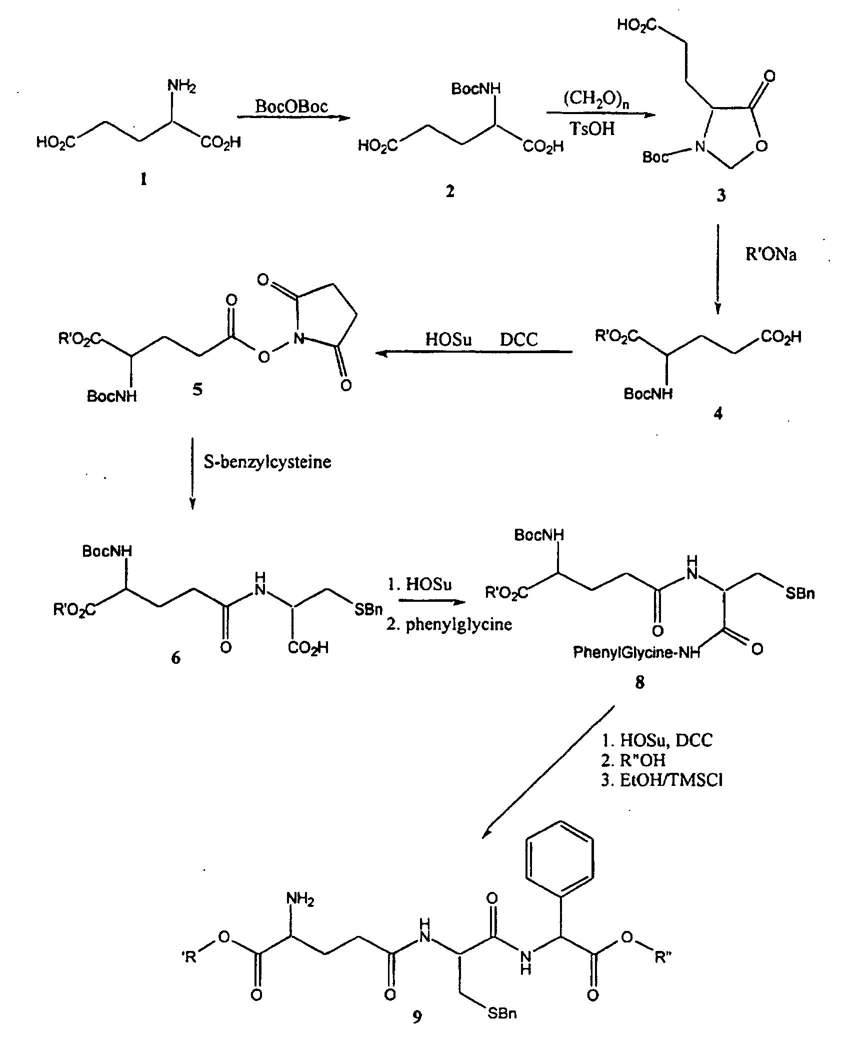 Intermediate 7 (N-a-t-Boc-a-ethyl-g-glutamyl-S(benzyl)cysteine succinimide ester) is not shown in the reaction scheme above.
p-toluenesulfonic acid. Compound **4** is prepared by the reaction of the oxazolidinone and R'ONa. The succiminide ester **5** is prepared with DCC coupling with N-hydroxysuccinimide and the g free acid **4**, which is then reacted with S-benzyl-L-cysteine for the formation of the protected dipeptide 6. The succinimide ester is prepared through a DCC coupling and its reaction with phenylglycine yields the protected tripeptide, **8.** Deprotection and esterification leads to final product **9**.

### Example 2

### N-α-t-Boc-L-glutamic acid, 2

Two 500g portions of L-glutamic acid (6.8 mols) were suspended in 4 L of 10% THF in water each. Na₂CO₃ powder was added to pH 9 resulting in a clear solution. Each solution was heated to 40°C (for the first 10 hrs during the addition of the Boc anhydride) and 1 kg of di-t-butyl dicarbonate (4.58 mols) was added as a solid in 50g increments to each flask every 30 min. while the pH was maintained at 9 by addition of Na₂CO₃. The reaction was stirred overnight at room temperature. The following day, an additional 1 kg of di-t-butyl dicarbonate was added as above. On the morning of the third day each solution was extracted twice with two 2 L each of EtOAc (It was found that the formation of the butanol stopped the reaction after the addition of 1 eq of the Boc anhydride and that the extraction with EtOAc removed it and increased the yield significantly). The aqueous layer was collected and 1 kg of di-t-butyl dicarbonate was again added as above (heating to 40°C and adding 500g/day in 50g increments to each flask over a period of two days). The total reaction time was four days with the addition of 4 kg (18.32 mols) of the di-t-butyl dicarbonate. The long reaction time and heating was found essential for acceptable yields. The reaction mixtures were then extracted twice with 2 L each of EtOAc. Each of the aqueous layers were collected, cooled to 0°C in an ice bath and acidified dropwise with conc. HCl over 30 min (1 drop/sec) to pH 3 (The BocGlu was found to be sensitive to this acidification step. If the pH was brought too low or if the HCl was added too fast, some deprotection occurred and large amounts of glutamic acid precipitated out of solution.). Upon complete acidification an off white gel formed, which was intensified by the liberal addition of NaCl to little beyond the solution's saturation point. The resulting solution was extracted twice with 2 L each of EtOAc. The removal of the Boc-L-Glu from the aqueous layer was monitored by TLC. The EtOAc layers were combined, dried over Na₂SO₄, filtered, and roto-evaporated at 35°C to a clear yellowish oil. This was dried *in vacuo* overnight to obtain 1097.1 g of BocGlu (4.44 mols); 65 % yield.

### (S)-t-Butyloxycarbonyl-5-oxo-4-oxazolidinepropanoic acid, 3

1077 g (4.36 mols) of Boc-L-Glu , 2, was dissolved in 1 L of CH₃CN with warming. This was poured into a 5 L three neck flask. To this solution 3 L of benzene, 196 g (6.54 mols) of paraformaldehyde, and 40 g (0.26 mols) of p-toluene sulfonic acid were added. Equipping the round bottom flasks with two Dean-Stark apparati, dual condensers at -10°C, and a mechanical stirrer, the reaction mixture was brought to 70°C and refluxed for 6 hours (100 ml of water was collected from the Dean-Stark apparati indicating complete reaction). The reaction mixtures, clear reddish-brown solutions, were allowed to cool to room temperature, and were rotoevaporated to a reddish-brown oil. The oil was redissolved in 2 L of ether and was extracted three times with 1 L each of water. The aqueous layers were combined and were extracted with 2 L of ether to remove the product which was in the water. Both ether layers were combined, dried over Na₂SO₄, filtered, and roto-evaporated to a clear yellow oil. This was dried *in vacuo* to obtain 952 g (3.67 mols), 84.3% yield of impure 3.

3 was purified using an open column made from a 3 L coarse sintered glass funnel modified with a 30 cm glass extension. 2 kg of Baker Silica Gel, 40um flash chromatography packing, was slurried with 4 L of CH₂Cl₂ and poured into the column. The solvent was allowed to drain until it reached the resin bed. An additional 2 L of CH₂Cl₂ were added and allowed to drain to the resin bed settling the silica. At this point, the compound dissolved (with gentle heating) in 750 ml of CH₂Cl₂ was poured into the column and the solvent allowed to drain. When the solvent level reached the resin bed an additional 1 L of CH₂Cl₂ was added and allowed to drain to complete the loading of the product. It was then eluted with 20 L of CH₂Cl₂ and collected in 400 ml fractions. Evaporation to an oil, and dried to a whitish-yellow foam under a high vacuum; 745.7 g (2.88 mols), 66.2% yield was obtained.

### N-α-t-Boc-L-Glutamic acid α-ethyl ester, 4

150 g of sodium sticks (6.5 mols) were placed into a container with 500 ml of toluene, the surface tarnish was scraped off, and thin squares were cut. A 50g portion of the squares were weighed into a tared container with 100 ml of toluene, the toluene was poured off and the sodium was added to 3 L of punctilious ethanol at 0°C under a stream of N₂. After the bubbling subsided, another 50g portion was prepared as above and added. This was repeated until the entire 200g was added. The solution was then stirred at room temperature under N₂ overnight resulting in a viscous clear solution. 745.7 g (2.88 mols) of 3 was dissolved into 3 L of punctilious EtOH (with warming) and placed into a 5 L, three necked round bottom flask equipped with a mechanical stirrer. To this solution, under a stream of N₂, using an equalizing dropping funnel, the NaOEt was added dropwise (1 drop/sec.). The reaction was stirred overnight to produce a yellowish-white solution with a large amount of a white precipitate. The reaction mixture was chilled to 0°C in an ice bath and conc. HCl was added dropwise (1 drop/sec.) to pH 9 (the pH was at 13 after the reaction). 6 L of water was added to produce a clear solution. This was extracted twice with 3 L each of a 1:1 mixture of ether: petroleum ether. The aqueous was collected, chilled to 0°C in an ice bath, and while stirring, HCl was added dropwise (1 drop/sec) to pH 3. The acidified solution was extracted twice with 3 L ether each. The ether layers were combined, dried over Na₂SO₄, filtered, and evaporated to a clear yellow oil. This was dried under a high-vacuum to give 609g (2.11 mols), 73.5% yield.

### N-α-t-Boc-L-Glutamic acid α-ethyl ester γ-succinimide ester, 5

609 g (2.11 mols) of N-α-t-Boc-L-Glutamic acid α-ethyl ester **4**, and 280.4 g of N-hydroxysuccinimide (2.43 mols) were dissolved in 3 L of CH₂Cl₂ in a 5 L, three neck round bottom, equipped with a mechanical stirrer. 547.1g of N,N'-dicyclohexylcarbodiimide (2.65 mols) was dissolved in 500 ml of CH₂Cl₂ and was added dropwise, with stirring, under a stream of N₂. An additional 50g of DCC (0.24 mol) was dissolved in 100 ml of CH₂Cl₂ and added dropwise to the reaction mixture and stirred overnight. TLC showed complete reaction. The DCC urea was filtered from the reaction mixture, and the filtrate was rotoevaporated to an oil and dried to a foam under a high vacuum; 1042.7g (2.81 mols) of crude 5 was obtained.

5 was purified using the open column used for the purification of 3. 1.5 kg of Baker Silica Gel, 40µm flash chromatography packing, was slurried in 4 L of CH₂Cl₂ and poured into the column. This was allowed to drain until the solvent level reached the resin bed. An additional 2 L of CH₂Cl₂ was added and allowed to drain to the resin bed in order to pack the silica. When the solvent level reached the top of the resin bed, the compound dissolved (with gentle heating) in 750 ml of CH₂Cl₂ was poured into the column and allowed to load by gravity. When the solvent reached the resin bed, 1 L of CH₂Cl₂ was added and allowed to drain in order to completely load the product. The product was eluted with 20 L of CH₂Cl₂ and collected in 400 ml aliquots. Fractions were evaporated to an oil, and dried to a foam under a high vacuum; 853 g (2.28 mols), 108.0% yield (***note purity in supplement* #5)** was obtained.

### N-α-t Boc-α-ethyl-γ-glutamyl-S(benzyl)-L-cysteine, 6

465g (2.2 mols) of S(benzyl)-L-cysteine was slurried in 4 L of a 20% THF and water solution. Na₂CO₃ powder was added to the solution while stirring magnetically to pH 9. This produces a partially dissolved slurry. 853g (2.28 mols) of 5 was dissolved in 1.5 L of THF with gentle heating. This was added dropwise (2 drop/sec) to the stirring solution while maintaining the pH at 9 with Na₂CO₃. The reaction mixture was extracted twice with 2 L each of 1:1 ether: petroleum ether. TLC was checked to ensure that the product remained in the aqueous phase due to the large amount of THF. The aqueous phase was separated and chilled to 0°C in an ice bath. Concentrated hydrochloric acid was added dropwise while the solution was stirred magnetically until the pH reached 3. The oily precipitate produced was then extracted into two 2 L portions of ether. The ether layers were combined, poured over Na₂SO₄, filtered, evaporated to an oil, and dried to a foam under a high vacuum giving 895.8 g (1.91 mols), 90.4% yield.

### N-α-t-Boc-α-ethyl-L-γ-glutamyl-L-S(benzyl)cysteine succinimide ester, 7

895 g (1.91 mols) of 6, and 240.6 g of N-hydroxysuccinimide (2.09 mols) were dissolved in 3 L of CH₂Cl₂ in a 5 L, three neck round bottom, equipped with a mechanical stirrer. 472.5 g of N,N'-dicyclohexylcarbodiimide (2.29 mols) was dissolved in 500 ml of CH₂Cl₂ and was added dropwise (2 drops/sec) with stirring, under a stream of N₂. After 5 hrs, a TLC showed a substantial amount of starting material. An additional 50g (0.24 mol) of DCC was dissolved in 50 ml of CH₂Cl₂ and added dropwise to the reaction mixture and stirred overnight. TLC showed complete reaction. The DCC urea was filtered from the reaction mixture, and the filtrate was evaporated to 1 L. Ether was added to cloud point and the solution was chilled to 0°C in an ice bath. Upon scratching crystals began to form. Small amounts of ether were added to maintain cloud point and push out the product. The solution was stored at 4°C overnight. The crystals were collected by filtration and dried under a high vacuum; 953 g (1.67 mols), 87.4% yield was obtained.

### N-α-t-Boc-α-ethyl-L-γ-glutamyl-L-S(benzyl)cystenyl-R-phenylglycine, 8

253 g (1.67 mols) of (R)-(-)-phenylglycine was slurried in 4 L of a 20% THF and water solution. Na₂CO₃ powder was added to the magnetically stirred solution to a pH of 9, producing a partially dissolved slurry. 953 g (1.67 mols) of 7 was dissolved in 1.5 L of THF with gentle heating. This was added dropwise (2 drop/sec) to the stirring solution while the pH was maintained at 9 by addition of Na₂CO₃. The reaction was stirred 6 hours resulting in a clear yellowish-orange solution. The reaction mixture was extracted twice with 2 L each of 1:1 ether: petroleum ether. TLC was checked to ensure that the product remained in the aqueous layer due to the large amount of THF. The aqueous layer was separated and chilled to 0°C in an ice bath. While magnetically stirring the solution, hydrochloric acid (conc.) was added dropwise (1 drop/sec) until the pH reached 3. The product was then extracted twice with 2 L each of ether. The ether layers were combined, poured over Na₂SO₄, filtered, evaporated to an oil, and dried to a foam under a high vacuum yielding 853 g (1.45 mols), 86.8% yield.

### γ-Glutamyl-S(benzyl)cysteinyl-R-phenyl glycine diethyl ester hydrochloride, 9

767.5g (1.26 mols) of 8 was divided into two portions and each dissolved in 2.4 L of punctilious EtOH in 5 L three neck round bottoms equipped with mechanical stirrers. Each solution was placed into an ice bath and chilled to 0°C while stirring under a stream of argon. 385 ml (6.32 mols) of (CH₃)₃SiCl was added dropwise (1 drop/sec) to each solution while the temperature was maintained at 0°C. After the (CH₃)₃SiCl was added (2.5 hrs), the reactions were taken out of their ice baths and allowed to warm to room temperature. The reactions are stirred overnight. After 18 hours, the reaction mixtures become a white solid mass. The solid is then broken up to form a slurry and each is rotoevaporated at 30°C to a volume of 2 L each. While stirring vigorously with the mechanical stirrers, 2 L of anhydrous ether is added dropwise (1 drop/sec.) to each slurry over a period of 2.5 hours at room temperature. The insoluble material was collected by filtration. The filter cakes are dried overnight under a high vacuum to yield a combined total of 312.8 g of 9. This 312.8 g was dissolved in 7 L of hot CH₃CN (79°C) with stirring, filtered hot to remove insoluble material, and the filtrate allowed to cool to room temperature. As the solution cooled, small white needle-like crystals began to form and when it reached room temperature it solidified to a solid mass. This was broken up and the solid was collected by filtration. The filter cake was dried 16 hours under a high vacuum over NaOH pellets. The dried material was to afford 277.0 g (0.49 mol), 38.8% yield was obtained. Compounds 10, 11, and 13 are obtained in a similar manner. Compound 12 is obtained from compound 8 following removal of the t-Boc group by cleavage with concentrated HCl.

**Table 1**

| **Description of Substituents R1, R2, and R3 in Compounds 9-13** | | | |
|---|---|---|---|
| **Compound** | **R1** | **R2** | **R3** |
| **9** | CH₂CH₃ | CH₂CH₃ | Benzyl |
| **10** | (CH₂)₇CH₃ | CH₂CH₃ | Benzyl |
| **11** | (CH₂)₁₅CH₃ | CH₂CH₃ | Benzyl |
| **12** | (CH₂)₁₅CH₃ | H | Benzyl |
| **13** | (CH₂)₉CH₃ | CH₂CH₃ | Benzyl |

### Example 3

### Preparation of Lipid Microspheres

30 mg of 9, 10g of purified soybean oil, and 1.8 g of purified yolk lecithin were added, and the mixture was melted under heating by means of a homogenizer. Then 2.21 gm of glycerol and enough distilled water to bring the volume to 100 mL(approximately 90 mL) were added, followed by a rough emulsification by means of a homogenizer. The product is then further emulsified by means of a manton gaulin type homogenizer. The solution is then passed through a 1.2µm filter to afford a final emulsion of approximately 220nM particles at 300 µg/mL. In a similar manner, lipid microspheres containing compounds 10 to 12 are prepared.

**Table 2**

| **Measured Characteristics of Lipid Microspheres** | | | | |
|---|---|---|---|---|
| **Compound** | **Conc. (mg/mL)** | **Particle Size** | **Incorporation of compound in Lipid Particle** | **Decrease of K**_{**1**} **in esterase hydrolysis** |
| | | Mean ±SD (nM) | Fraction of 1.2 µm-filter passing (%) | |
| 9 | 0.1-1 | | 93.7 | 0.609 |
| 10 | 0.03-3 | 220.7±106. 3 | 96.8-1-5.9 | 0.454 |
| 11 | 1-3 | 219.6±76.0 | 90.6-91.3 | 0.185 |
| 12 | 0.3 | 222.3±76.4 | 91.9 | |

The decrease of K₁ reported in Table 2 above is an indicator of enhanced bioavailability of the glutathione analogs of the invention. Variations and modifications to the above described invention are, of course, possible. Accordingly, the present invention is not limited to the embodiments described in detail above.

### Example 4 Liposome Manufacturing Process

Unless otherwise stated, the following general procedure is used to manufacture the lipid formulation: (i) 1573 mg compound 9, 3146 mg of egg phosphatidylcholine (EPC), and 3146 mg of egg phosphatidylglycerol (EPG), are dissolved in 14.3 ml anhydrous ethanol and 2.2 ml H₂O by mixing at ambient temperature; (ii) the solution is taken up in a 30 ml syringe using a 20 gauge needle and injected directly (within 1 minute) into 80.9 ml of stirred 370 mM sucrose to form multilamellar vesicles (MLVs); (iii) 2.5 ml of 1 M citrate (pH 4.0) is added to the stirred buffer; (iv) the solution is extruded ten times through two stacked 80 nm polycarbonate filters using a 100 ml EXTRUDER (Lipex Biomembranes, Vancouver Canada) to form unilamellar vesicles; (v) passed through 0.22 micron filters to sterilize; and (vi) lyophilized to remove water and ethanol. The lyophilization cycle involved rapid freezing of the sample (2.5 ml fill of 10 ml vial) to a sample temperature of -50°C, followed by lyophilization at a tray temperature of -35°C for 42 hours to remove primary water, and for 2 hours at 20°C to remove secondary water.

Quasi Elastic light Scattering (QELS) on the samples was carried out to determine the average vesicle size using a Nicomp Particle Sizer (Model 270). The level of drug incorporation (% encapsulation) in the vesicles is determined using two independent approaches. In the first approach, 0.1 ml of formulation is placed on a 1.0 ml tuberculin syringe containing hydrated sephadex G-50; the sample is centrifuged for 5 min. at 2,000 rpm in a tabletop Centrifuge and the vesicles are recovered (>70%) in the void volume. Unencapsulated or precipitated drug is removed by this process, and remains on the gel filtration column. The level of encapsulation (%) is calculated from the drug/lipid ratio prior to and following column chromatography. A second approach relies upon separation of the liposomes from unencapsulated drug by centrifugation through Micron30 filters with a MW cutoff of 30,000 Daltons (Amicon). The level of encapsulation (%) is calculated from 1- ([Free Drug]/[Total drug]) * 100.

### Example 5 Formulation of 9 in lipids using dilution from Ethanol

The results presented in Table 3 below summarize the incorporation of 9 in several lipid formulations as a function of drug/lipid ratio and lipid composition. Dilution of the ethanol solution into a sucrose buffer results in the spontaneous formation of liposomes (termed multilamellar vesicles, MLVs), which are relatively large (average diameter of 700 nm), heterogeneous, but do not contain visible lipid or drug precipitates. The MLVs may be passed (5-10x) through stacked polycarbonate filters (pore size of 80 nm) using an EXTRUDER (Lipex Biomembranes, Vancouver, Canada). This process removes the precipitated form of the drug (< 2%), and reduces the vesicle size to an average diameter of 140 nm (+/- 20 nm).

**Table 3**

| Incorporation of Compound 9 into Liposomes by Ethanol Dilution | | | | |
|---|---|---|---|---|
| Drug/Lipid (wt/wt) | Lipid Composition | Drug (mg/ml) | Encapsulation (Microcon, %) | Size (nm) |
| 0.25¹ | EPC | 16.2. | 56 | 69 (+/- 22) |
| 0.1¹ | EPC | 12.9 | 80 | 101 (+/- 40) |
| 0.25¹ | EPC/EPG (9/1) | 13.6 | 60 | 69 (+/- 19) |
| 0.14¹ | EPC/EPG (2.2/1) | ND | ND | ND |
| 0.14 | EPC/EPG (1/1) | 14.4 | 99 | 92 |
| ND signifies not determined. | | | | |

| | | | | |
|---|---|---|---|---|
| ¹ comparative example | | | | |

### Example 6 Effect of lyophilization and vesicle size

The results presented in Table 4 below summarize the relation between lyophilization conditions and the resulting vesicle size, and level of drug encapsulation. Unless otherwise seated, vesicles were prepared as described in Example 4, sterile filtered and vialed in 2.5 ml aliquots. The vials were subsequently frozen to a sample temperature of -50°C for sufficient time to reduce the sample temperature to the shelf temperature. The samples were then exposed to the indicated shelf temperature in the presence of a vacuum (50 to 200 mTorr) for sufficient time to remove the primary water (24 to 48 hours), and subsequently to ambient temperature for 2-6 hours.

**Table 4**

| The Influence of Different Lyophilization Cycles on Compound 9 Liposomes | | | | | |
|---|---|---|---|---|---|
| Drug/Lipid (wt/wt) | Lipid Composition | Sucrose (mM) | Lyophilization Conditions | Encapsulation (Microcon, %) | Size (nm) |
| 0.25 | EPC¹ | 300 | Uncontrolled | 73 | 2844 |
| 0.14 | EPC¹ | 300 | Uncontrolled | 87 | 8871 |
| 0.14 | EPC¹ | 700 | -10°C | 85% | NA |
| 0.14 | EPC/EPG (1/1) | 300 | -10°C | 99 | 1418 |
| 0.14 | EPC/EPG (1/1) | 700 | -10°C | 98 | 535 |
| 0.14 | EPC/EPG (1/1) | 700 | -30°C | 99 | 491 |

| | | | | | |
|---|---|---|---|---|---|
| ¹ comparative example | | | | | |

### Example 7 Influence of Drug/Lipid Ratio on Encapsulation Level

The results provided in Table 5 below summarize the influence of the initial drug/lipid ratio on the level of encapsulation. Compound 9 can. be efficiently encapsulated in EPC/EPG liposomes at drug/lipid ratio of up to 0.33 (wt/wt).

**Table 5**

| Encapsulation of Compound 9 in EPC/EPG Liposomes, as a Function of Drug/Lipid Ratio | | | | | |
|---|---|---|---|---|---|
| Drug/Lipid (wt/wt) | Lipid Composition | Sucrose (mM) | Lyophilization Conditions | Encapsulation (Microcon, %) | Size (nm) |
| 0.50 | EPC/EPG (1/1) | 300 | Uncontrolled | ND | ND |
| 0.33 | EPC/EPG (1/1) | 300 | Uncontrolled | 99 | 1402 |
| 0.25 | EPC/EPG (1/1) | 300 | Uncontrolled | 100 | 715 |
| 0.20 | EPC/EPG (1/1) | 300 | Uncontrolled | 100 | 750 |

### Example 8 Vesicle Size Stability

For all batches examined, the level of encapsulation was not influenced by storage at 2-8°C for up to 48 hours as shown in Table 6 below.

**Table 6**

| **Stability of Vesicles Size Following Extrusion, as a Function of Ethanol Content** | | | | | | |
|---|---|---|---|---|---|---|
| Drug/Lipid (wt/wt) | Lipid Composition | Ethanol (%) | 0 hr | 2 hr | 24 hr | 48 hour |
| 0.25 | EPC/EPG (1/1) | 15 | 161 nm | 156 nm | 179 nm | 160 nm |
| 0.25 | EPC/EPG (1/1) | 20 | 120 nm | 123 nm | 273 nm | ND |

### Example 9 Effect of Lyophylization Time on Encapsulation

The results presented in Table 7 below summarize the influence of lyophilization conditions on the encapsulation efficiency, viscosity, and size of a compound 9 EPC/EPG formulation.

**Table 7**

| **The Influence of Primary Lyophilization Cycle on Encapsulation, Vesicle Size and Viscosity of Compound 9 EPC/EPG Formulations** | | | | | | |
|---|---|---|---|---|---|---|
| Drug/Lipid (wt/wt) | Lipid Composition | Sucrose (mM) | Lyophilization Time (hr) | Encapsulation (Microcon, %) | Size (nm) | Viscosity |
| 0.25 | EPC/EPG (1/1) | 300 | 32 | 99 | 611 | Negligible |
| 0.25 | EPC/EPG (1/1) | 300 | 40 | 99 | 391 | Negligible |
| 0.25 | EPC/EPG (1/1) | 300 | 44 | 97 | 584 | Negligible |
| 0.25 | EPC/EPG (1/1) | 300 | 54 | 98 | 502 | Viscous |
| 0.25 | EPC/EPG (1/1) | 300 | 59 | 98 | 636 | Very Viscous |

### Example 10 Rat Model of chemotherapy induced neutropenia

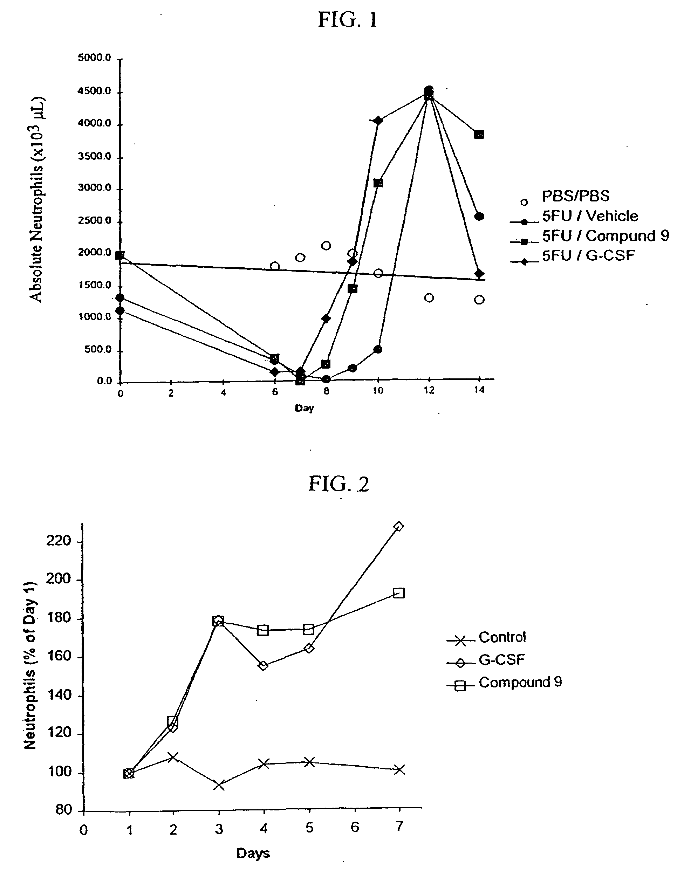

Compound 9 formulated as described in Example 4 accelerated recovery from chemotherapy-induced neutropenia in rats. Male CD rats (250-300g) were injected intraperitoneally with either the antineoplastic drug, fluorouracil (125mg/kg) or an equivalent volume of its vehicle (saline). Beginning one day after the fluorouracil injection, each fluorouracil-treated rat was given 8 daily intravenous injections of either Compound 9 (35mg/kg, n = 4), G-CSF (10µg/kg, n = 5), or vehicle (saline, n = 4). Rats originally injected with saline rather than fluorouracil received saline intravenously (n = 7). Blood samples were taken for measurement of neutrophil levels prior to the fluorouracil and every 1 to 2 days thereafter for 14 days. The accelerated recovery from fluorouracil-induced neutropenia by lyposomally-formulated Compound 9 is shown in Figure 1.

### Example 11 Effect of Compound 9 on normal rats

Compound 9 as described in Example 4 (35mg/kg, i.v.) also increased circulating neutrophil levels in normal rats (figure B). Male, CD rats (250-300g) were given 5 daily intravenous injections of either Compound 9 (35mg/kg, n = 9), G-CSF (10 µg/kg, n=4), or vehicle (saline, n = 11). Blood samples were taken for measurement of neutrophil levels daily. The elevation of circulating neutrophil levels by lipid-formulated Compound 9 in the normal rat is shown in Figure 2.

## Claims

1. A lipid formulation containing a compound that is:
(i) a diester of a compound of formula A 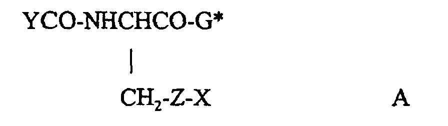 wherein:
each ester is 1-25C;
YCO is γ-glu or β-asp;
G* is phenylglycine;
Z is CH₂, O or S; and
X is a hydrocarbon radical which is alkyl (6-8C), benzyl, or naphthyl;
or a pharmaceutically acceptable salt thereof; or
(ii) a compound of formula I 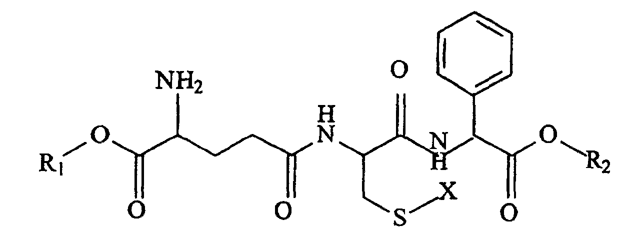 wherein:
R₁ and R₂ are each independently linear or branched alkyl (1-25C), cycloalkyl (6-25C), heterocycle (6-20C), ether or polyether (3-25C), or R₁-R₂ together have 2-20 C atoms and form a macrocycle with the remainder of formula I; and
X is as defined above for formula A;
or a pharmaceutically acceptable salt thereof;
wherein the lipids of said lipid formulation comprise egg phosphatidylcholine and egg phosphatidylglycerol in a ratio of 0.75-1.25:0.75-1.25 by weight.

2. The lipid formulation of claim 1 where the compound is γ-glutamyl-S(benzyl)cysteinyl-R-phenylglycine diethyl ester or a pharmaceutically acceptable salt thereof.

3. The lipid formulation of claims 1 or 2 where the ratio of lipids to compound is 3.5-4.5:0.5-1.5 by weight.

4. The lipid formulation of claim 3 where the ratio of lipids to compound is 3:1-6:1 by weight.

5. The lipid formulation of any one of claims 1 to 4 where the formulation is a liposomal formulation.

6. The lipid formulation of any one of claims 1 to 5, having
(i) at least 50% degree of encapsulation of the compound; and
(ii) an average vesicle size of 50 - 2000 nm

7. The lipid formulation of claim 6 where the degree of encapsulation is above 80%.

8. The lipid formulation of claims 6 or 7 where the vesicle size is 400 - 600 nm.

9. The lipid formulation of any one of claims 1 to 8 which is a liposomal formulation composed of 1 part compound, 2 parts egg phosphatidylcholine, 2 parts egg phosphatidylglycerol, and 7 parts sucrose by weight.

10. The lipid formulation of claim 9 which comprises lyophilized liposomes composed of 1 part γ-glutamyl-S(benzyl)cysteinyl-R-phenylglycine diethyl ester or a pharmaceutically acceptable salt thereof, 2 parts egg phosphatidylcholine, 2 parts egg phosphatidylglycerol, and 7 parts sucrose by weight.

11. The lipid formulation of any one of claims 1 to 10 which is prepared by extrusion.

12. A method of preparing a lipid formulation containing a compound that is:
(i) a diester of a compound of formula A 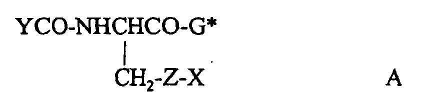 wherein:
each ester is 1-25C;
YCO is γ-glu or β-asp;
G* is phenylglycine;
Z is CH₂, O or S; and
X is a hydrocarbon radical which is alkyl (6-8C), benzyl, or naphthyl;
or a pharmaceutically acceptable salt thereof; or
(ii) a compound of formula I 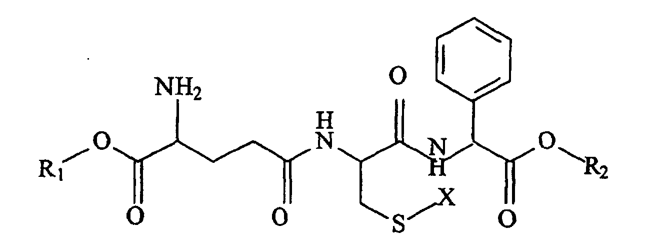 wherein:
R₁ and R₂ are each independently linear or branched alkyl (1-25C), cycloalkyl (6-25C), heterocycle (6-20C), ether or polyether (3-25C), or R₁-R₂ together have 2-20 C atoms and form a macrocycle with the remainder of formula I; and
X is as defined above for formula A;
or a pharmaceutically acceptable salt thereof;
which method comprises formulating the compound in a lipid composition comprising egg phosphatidylcholine and egg phosphatidylglycerol in a ratio of 0.75-1.25:0.75-1.25 by weight.

13. The method of claim 12 where the compound is γ-glutamyl-S(benzyl)cysteinyl-R-phenylglycine diethyl ester or a pharmaceutically acceptable salt thereof.

14. The method of claims 12 or 13 where the formulation is a liposomal formulation.

15. The method of any one of claims 12 to 14, further comprising extrusion.

16. The method of any one of claims 12 to 15, further comprising lyophilization.

17. The method of claim 16 which comprises dissolving 1 part γ-glutamyl-S(benzyl)cysteinyl-R-phenylglycine diethyl ester or a pharmaceutically acceptable salt thereof, 2 parts egg phosphatidycholine, and 2 parts egg phosphatidylglycerol in ethanol/water, injecting the solution into water containing 7 parts sucrose, extruding to form a liposomal formulation, and lyophilizing the liposomal formulation to form lyophilized liposomes.

18. The use of the lipid formulation of any one of claims 1 to 11 in the manufacture of a medicament for the modulation of hematopoiesis and protection against the destructive effects of chemotherapy.

## Patentansprüche

1. Lipid-Formulierung enthaltend eine Verbindung, welche ist:
(i) ein Diester einer Verbindung der Formel A 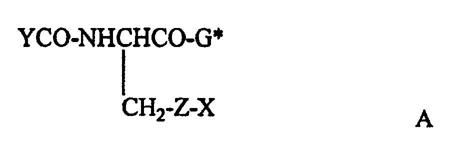 in der:
jeder Ester 1 - 25 C ist;
YCO für γ-Glu oder β-Asp steht;
G* Phenylglycin ist;
Z für CH₂, O oder S steht; und
X ein Kohlenwasserstoffrest ist, der Alkyl (6 - 8 C), Benzyl oder Naphthyl ist;
oder ein pharmazeutisch annehmbares Salz derselben; oder
(ii) eine Verbindung der Formel I 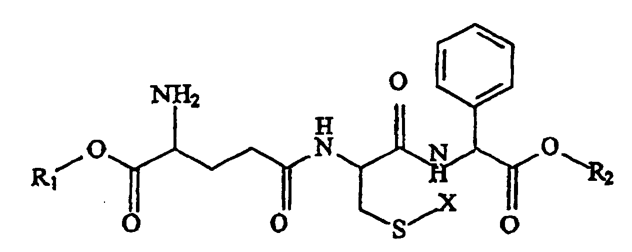 in der:
R₁ und R₂ jeweils unabhängig lineares oder verzweigtes Alkyl (1 - 25 C), Cycloalkyl (6 - 25 C), Heterocyclus (6 - 20 C), Ether oder
Polyether (3 - 25 C) sind oder R₁-R₂ zusammen 2 - 20 C-Atome aufweisen und einen Makrocyclus mit dem Rest der Formel I bilden; und
X wie oben für die Formel A definiert ist;
oder ein pharmazeutisch annehmbares Salz derselben;
wobei die Lipide der Lipid-Formulierung Ei-Phosphatidylcholin und Ei-Phosphatidylglycerin in einem Gewichtsverhältnis von 0,75 - 1,25:0,75 - 1,25 umfassen.

2. Lipid-Formulierung nach Anspruch 1, in der die Verbindung γ-Glutamyl-S(benzyl)cysteinyl-R-phenylglycindiethylester oder ein pharmazeutisch annehmbares Salz desselben ist.

3. Lipid-Formulierung nach Anspruch 1 oder 2, in der das Gewichtsverhältnis von Lipiden zu Verbindung 3,5 - 4,5:0,5 - 1,5 beträgt.

4. Lipid-Formulierung nach Anspruch 3, in der das Gewichtsverhältnis von Lipiden zu Verbindung 3:1 - 6:1 beträgt.

5. Lipid-Formulierung nach irgendeinem der Ansprüche 1 bis 4, in der die Formulierung eine liposomale Formulierung ist.

6. Lipid-Formulierung nach irgendeinem der Ansprüche 1 bis 5, mit
(i) einem mindestens 50%-igen Einkapselungsgrad der Verbindung; und
(ii) einer durchschnittlichen Vesikelgröße von 50 - 2000 nm.

7. Lipid-Formulierung nach Anspruch 6, in der der Einkapselungsgrad über 80% liegt.

8. Lipid-Formulierung nach den Ansprüchen 6 oder 7, in der die Vesikelgröße 400 - 600 nm beträgt.

9. Lipid-Formulierung nach irgendeinem der Ansprüche 1 bis 8, die eine liposomale Formulierung ist, die aus 1 Gewichtsteil Verbindung, 2 Gewichtsteilen Ei-Phosphatidylcholin, 2 Gewichtsteilen Ei-Phosphatidylglycerin und 7 Gewichtsteilen Saccharose zusammengesetzt ist.

10. Lipid-Formulierung nach Anspruch 9, die lyophilisierte Liposomen umfasst, welche aus 1 Gewichtsteil γ-Glutamyl-S(benzyl)cysteinyl-R-phenylglycindiethylester oder einem pharmazeutisch annehmbaren Salz desselben, 2 Gewichtsteilen Ei-Phosphatidylcholin, 2 Gewichtsteilen Ei-Phosphatidylglycerin und 7 Gewichtsteilen Saccharose zusammengesetzt sind.

11. Lipid-Formulierung nach irgendeinem der Ansprüche 1 bis 10, die durch Extrusion hergestellt wird.

12. Verfahren zur Herstellung einer Lipid-Formulierung, die eine Verbindung enthält, welche:
(i) ein Diester einer Verbindung der Formel A 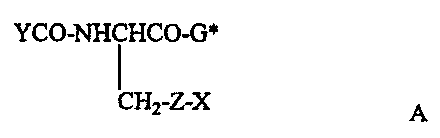 in der:
jeder Ester 1 - 25 C ist;
YCO für γ-Glu oder β-Asp steht;
G* Phenylglycin ist;
Z für CH₂, O oder S steht; und
X ein Kohlenwasserstoffrest ist, der Alkyl (6 - 8 C), Benzyl oder Naphthyl ist;
oder ein pharmazeutisch annehmbares Salz derselben; oder
(ii) eine Verbindung der Formel I 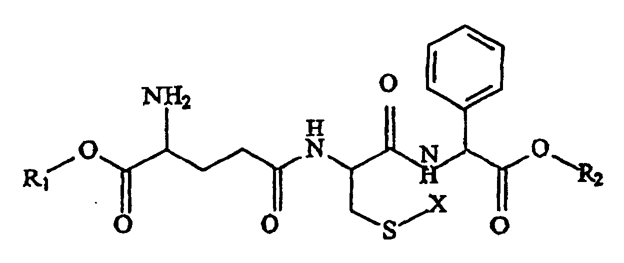 in der:
R₁ und R₂ jeweils unabhängig lineares oder verzweigtes Alkyl (1 - 25 C), Cycloalkyl (6 - 25 C), Heterocyclus (6 - 20 C), Ether oder
Polyether (3 - 25 C) sind oder R₁-R₂ zusammen 2 - 20 C-Atome aufweisen und einen Makrocyclus mit dem Rest der Formel I bilden; und
X wie oben für die Formel A definiert ist;
oder ein pharmazeutisch annehmbares Salz derselben;
ist, wobei das Verfahren umfasst, dass man die Verbindung in eine Lipid-Zusammensetzung formuliert, die Ei-Phosphatidylcholin und Ei-Phosphatidylglycerin in einem Gewichtsverhältnis von 0,75 - 1,25:075 - 1,25 umfasst.

13. Verfahren nach Anspruch 12, in dem die Verbindung γ-Glutamyl-S(benzyl)-cysteinyl-R-phenylglycindiethylester oder ein pharmazeutisch annehmbares Salz desselben ist.

14. Verfahren nach Anspruch 12 oder 13, in dem die Formulierung eine liposomale Formulierung ist.

15. Verfahren nach irgendeinem der Ansprüche 12 bis 14, weiter umfassend eine Extrusion.

16. Verfahren nach irgendeinem der Ansprüche 12 bis 15, weiter umfassend eine Lyophilisierung.

17. Verfahren nach Anspruch 16, welches umfasst, dass man 1 Teil γ-Glutamyl-S(benzyl)cysteinyl-R-phenylglycindiethylester oder ein pharmazeutisch annehmbares Salz desselben, 2 Teile Ei-Phosphatidylcholin und 2 Teile Ei-Phosphatidylglycerin in Ethanol/Wasser löst, die Lösung in Wasser einspritzt, das 7 Teile Saccharose enthält, extrudiert, um eine liposomale Formulierung zu bilden, und die liposomale Formulierung lyophilisiert, um lyophilisierte Liposomen zu bilden.

18. Verwendung der Lipid-Formulierung nach irgendeinem der Ansprüche 1 bis 11 bei der Herstellung eines Medikaments für die Modulation der Hämatopoese und zum Schutz gegen die destruktiven Auswirkungen einer Chemotherapie.

## Revendications

1. Formulation lipidique comprenant un composé qui est :
(i) un diester d'un composé de formule A 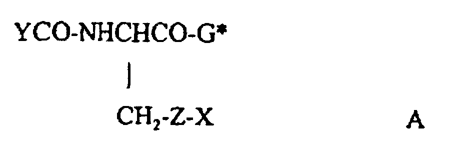 dans laquelle :
chaque ester est un groupe en C₁ à C₂₅ ;
YCO représente un groupe γ-glu ou β-asp ;
G* représente la phénylglycine ;
Z représente un groupe CH₂ ou un atome de O ou S ; et
X représente un radical hydrocarboné qui est un radical alkyle en C₆ à C₈, benzyle ou naphtyle ;
ou un de ses sels pharmaceutiquement acceptables ; ou
(ii) un composé de formule I 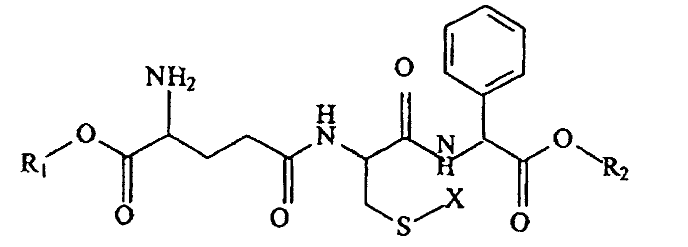 dans laquelle :
R₁ et R₂ représentent chacun indépendamment un groupe alkyle linéaire ou ramifié en C₁ à C₂₅, cycloalkyle en C₆ à C₂₅, hétérocyclique en C₆ à C₂₀, éther ou polyéther en C₃ à C₂₅, ou bien R₁ et R₂, conjointement, ont 2 à 20 atomes de carbone et forment un macrocycle avec le reste de la formule I ; et
X répond à la définition précitée pour la formule A ;
ou un de ses sels pharmaceutiquement acceptables ;
dans laquelle les lipides de ladite formulation lipidique comprennent de la phosphatidylcholine d'oeuf et du phosphatidylglycérol d'oeuf en un rapport de 0,75-1,25:0,75-1,25 en poids.

2. Formulation lipidique suivant la revendication 1, dans laquelle le composé est l'ester diéthylique de γ-glutamyl-S(benzyl)cystéinyl-R-phénylglycine ou un de ses sels pharmaceutiquement acceptables.

3. Formulation lipidique suivant la revendication 1 ou 2, dans laquelle le rapport des lipides au composé est égal à 3,5-4,5:0,5-1,5 en poids.

4. Formulation lipidique suivant la revendication 3, dans laquelle le rapport des lipides au composé est égal à 3:1-6:1 en poids.

5. Formulation lipidique suivant l'une quelconque des revendications 1 à 4, la formulation étant une formulation liposomale.

6. Formulation lipidique suivant l'une quelconque des revendications 1 à 5, ayant
(i) un degré d'encapsulation d'au moins 50 % du composé ; et
(ii) un diamètre moyen de vésicule de 50 à 2000 nm.

7. Formulation lipidique suivant la revendication 6, dans laquelle le degré d'encapsulation est supérieur à 80 %.

8. Formulation lipidique suivant la revendication 6 ou 7, dans laquelle le diamètre de vésicule est compris dans l'intervalle de 400 à 600 nm.

9. Formulation lipidique suivant l'une quelconque des revendications 1 à 8, qui est une formulation liposomale constituée de 1 partie du composé, de 2 parties de phosphatidylcholine d'oeuf, de 2 parties de phosphatidylglycérol d'oeuf et de 7 parties de saccharose, en poids.

10. Formulation lipidique suivant la revendication 9, qui comprend des liposomes lyophilisés constitués de 1 partie d'ester diéthylique de γ-glutamyl-S(benzyl)cystéinyl-R-phénylglycine ou d'un de ses sels pharmaceutiquement acceptables, de 2 parties de phosphatidylcholine d'oeuf, de 2 parties de phosphatidylglycérol d'oeuf et de 7 parties de saccharose, en poids.

11. Formulation lipidique suivant l'une quelconque des revendications 1 à 10, qui est préparée par extrusion.

12. Procédé pour la préparation d'une formulation lipidique contenant un composé qui est :
(i) un diester d'un composé de formule A 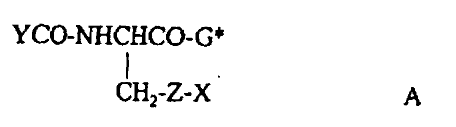 dans laquelle :
chaque ester est un groupe en C₁ à C₂₅ ;
YCO représente un groupe γ-glu ou β-asp ;
G* représente la phénylglycine ;
Z représente un groupe CH₂ ou un atome de 0 ou S ; et
X représente un radical hydrocarboné qui est un radical alkyle en C₆ à C₈, benzyle ou naphtyle ;
ou un de ses sels pharmaceutiquement acceptables ; ou
(ii) un composé de formule I 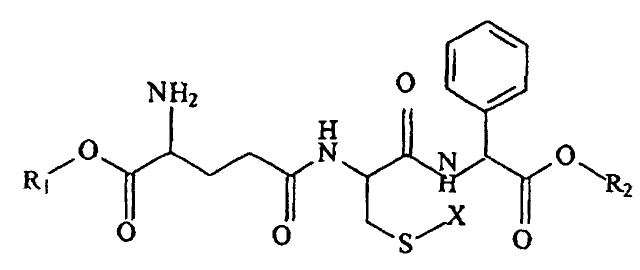 dans laquelle :
R₁ et R₂ représentent chacun indépendamment un groupe alkyle linéaire ou ramifié en C₁ à C₂₅, cycloalkyle en C₆ à C₂₅, hétérocyclique en C₆ à C₂₀, éther ou polyéther en C₃ à C₂₅, ou bien R₁ et R₂, conjointement, ont 2 à 20 atomes de carbone et forment un macrocycle avec le reste de la formule I ; et
X répond à la définition précitée pour la formule A ;
ou un de ses sels pharmaceutiquement acceptables ;
procédé qui comprend la formulation du composé dans une composition lipidique comprenant de la phosphatidylcholine d'oeuf et du phosphatidylglycérol d'oeuf en un rapport de 0,75-1,25:0,75-1,25 en poids.

13. Procédé suivant la revendication 12, dans lequel le composé est l'ester diéthylique de γ-glutamyl-S(benzyl)-cystéinyl-R-phénylglycine ou un de ses sels pharmaceutiquement acceptables.

14. Procédé suivant la revendication 12 ou 13, dans lequel la formulation est une formulation liposomale.

15. Procédé suivant l'une quelconque des revendications 12 à 14, comprenant en outre une extrusion.

16. Procédé suivant l'une quelconque des revendications 12 à 15, comprenant en outre une lyophilisation.

17. Procédé suivant la revendication 16, qui comprend la dissolution de 1 partie d'ester diéthylique de γ-glutamyl-S(benzyl)cystéinyl-R-phénylglycine ou d'un de ses sels pharmaceutiquement acceptables, de 2 parties de phosphatidylcholine d'oeuf et de 2 parties de phosphatidylglycérol d'oeuf dans un mélange éthanol/eau, l'injection de la solution dans de l'eau contenant 7 parties de saccharose, une extrusion pour former une formulation liposomale et une lyophilisation de la formulation liposomale pour former des liposomes lyophilisés.

18. Utilisation de la formulation lipidique suivant l'une quelconque des revendications 1 à 11 dans la production d'un médicament destiné à la modulation de l'hématopoïèse et à une protection contre les effets destructeurs de la chimiothérapie.
